# EUROPEAN PATENT APPLICATION

(11) **EP 4 556 470 A1**
(43) Date of publication of application: **21.05.2025**
(21) Application number: 23839008.2
(22) Date of filing: 13.07.2023
(51) Int. Cl.: C07D 307/46, B01J 19/00

(54) **PRODUCTION PROCESS AND DEVICE FOR CONTINUOUSLY PRODUCING 5-HYDROXYMETHYLFURFURAL**

(30) Priority: 14.07.2022 CN 202210826545
(71) Applicant: Zhongke Guosheng (Hangzhou) Technology Co., Ltd, Hangzhou, Zhejiang 310051 (CN)
(72) Inventor: WANG, Yantao, Hangzhou, Zhejiang 310051 (CN); ZHANG, Yu, Hangzhou, Zhejiang 310051 (CN); LIANG, Yue, Hangzhou, Zhejiang 310051 (CN); WANG, Bo, Hangzhou, Zhejiang 310051 (CN)
(74) Representative: Bandpay & Greuter
(86) International application number: PCT/CN2023/107171
(87) International publication number: WO 2024/012518

(57) **Abstract**

The present invention relates to the fields of catalytic chemistry and biomass resource utilization, particularly to a production process and device for continuously producing 5-hydroxymethylfurfural. The production process uses sugar biomass, a solvent, an auxiliary agent, and an acid catalyst as raw materials, and mixes them uniformly to obtain a water phase material flow. The water phase material flow is preheated and enters a multi-stage reaction apparatus. The product obtained from the reaction is cooled and separated to obtain 5-hydroxymethylfurfural. The device of the present invention takes into account both pure water phase and two-phase system, adopts fully automated program to control the production process, is provided with an online monitoring apparatus, and can monitor the production status of HMF in real time; At the same time, it can effectively solve the problem of the black rot easily blocking pipes caused by the generation of a large amount of humins in the process of producing 5-hydroxymethylfurfural, and the production efficiency is high. The device has broad development prospects for the large-scale industrial production of 5-hydroxymethylfurfural in the future.

## Description

### TECHNICAL FIELD

The present invention relates to the fields of catalytic chemistry and biomass resource utilization, particularly to a production process and device for continuously producing 5-hydroxymethylfurfural.

### BACKGROUND

5-hydroxymethylfurfural (HMF), as an important intermediate compound connecting biomass and chemical raw materials, can be synthesized through chemical reactions such as hydrogenation, oxidation, etherification, esterification to produce a series of other novel furan derivatives with high added value, such as 2,5-furandicarboxylic acid, 2,5-tetrahydrofuran dimethyl alcohol. It has great application prospects and value.

At present, the main methods for preparing 5-hydroxymethylfurfural are: (1) it is obtained by direct dehydration of fructose; (2) it is obtained by isomerization of glucose followed by dehydration; (3) 5-hydroxymethylfurfural is synthesized by cellulose hydrolysis. However, in the synthesis process of 5-hydroxymethylfurfural, there are many accompanying Humins, and their polymerization forms are complex, such as etherification reactions between HMF molecules, acetalization reactions between HMF molecules, and esterification reactions between HMF and acetic acid, formic acid, etc. The corresponding black rot presents various forms such as fine soft particles, hard particles, soft clumps, and loose flocs. In the high-temperature reaction stage, the solubility of black rot is relatively high. After being cooled down, the solubility of high polymers decreases, and most of them precipitate, deposit or adhere in the form of solid or semi-solid black rot. Especially in pure water phase systems, the increase in water amount makes the humins structure very dense, appearing as black hard blocks, which can easily cause pipeline blockage and make it difficult to dredge the pipeline. In intermittent kettle reactions, the impact of black rot on the production process is relatively small, while in the development of continuous flow reaction processes, there will be many challenges. For example, the reaction and the preparation of HMF are conducted on the traditional continuous fixed bed, and the generated black rot is easily adhered to the catalyst surface, reducing catalyst activity and even causing catalyst deactivation. On the other hand, the accumulation of this black rot for a long time may further cause complete blockage of pipelines, bends, valves, etc., leading to the inability to produce. Therefore, the problem of the black rot not blocking the pipes is an urgent issue to be solved in achieving continuous production of HMF.

In view of this, the present invention provides a production process and device for continuously producing 5-hydroxymethylfurfural with respect to the shortcomings of the existing technologies mentioned above.

### SUMMARY

### (1) The technical problem to be solved

The object of the present invention is to provide a production process and device for continuously producing 5-hydroxymethylfurfural, in order to solve the problem of easy blockage of pipelines and difficulty in continuous production in the process of producing 5-hydroxymethylfurfural.

### (2) Technical solution

In order to solve the above problem, in a first aspect, the present invention provides a device for continuously producing 5-hydroxymethylfurfural, comprising:
a material conveying system including a water phase material flow conveying unit for conveying the water phase material flow;
a first flushing flow system for cleaning during blockage and discharge, the first flushing flow system and the water phase material conveying unit being connected to the outlet of the material conveying system respectively;
a heat source system for heating the material, the heat source system being connected to the outlet of the material conveying system through a first pipeline;
a modular reaction apparatus system connected to the outlet of the heat source system through a second pipeline, the modular reaction apparatus system being built-in with a material reaction pipeline unit, a temperature sensing control unit, and a pressure sensing control unit;
a cooling system for cooling products in the modular reaction apparatus system, the cooling system being connected to the outlet of the modular reaction apparatus system through a third pipeline;
a control system, the water phase material flow conveying unit, the first flushing flow system, the heat source system, the temperature sensing control unit, the pressure sensing control unit, and the cooling system being connected to the control system respectively, for intelligent control of feeding, apparatus automatic flushing, temperature, pressure, emergency start, and emergency stop.

Furthermore, the material conveying system further includes an organic phase material flow conveying unit for conveying the organic material flow and the organic phase material flow conveying unit is connected to the outlet of the material conveying system.

Furthermore, the modular reaction apparatus system includes a plurality of modular reaction apparatuses. The temperature sensing control unit is a temperature sensor, and the pressure sensing control unit is a pressure sensor. The modular reaction apparatus includes a material reaction pipeline unit, a temperature sensor, and a pressure sensor that are built-in. The interior of the material reaction pipeline unit is entirely lined with polytetrafluoroethylene inner pipe, and the interior of the material reaction pipeline unit is built-in with a temperature sensor and a pressure sensor, which are respectively connected to the control system. The material reaction pipeline unit is provided with a heating element connected to the control system.

Furthermore, a second flushing flow system is included further. The plurality of modular reaction apparatuses are sequentially arranged in series or parallel. The modular reaction apparatuses further includes a first flushing three-way valve and a second flushing three-way valve. The first flushing three-way valve and the second flushing three-way valve are respectively provided at the position near the inlet of the material reaction pipeline unit and the position near the outlet of the material reaction pipeline unit. The first flushing three-way valve is connected to the second flushing flow system, and the first flushing three-way valve and the second flushing three-way valve are respectively connected to the control system.

Furthermore, the online monitoring system includes a UV online detector and a sampling probe connected to each other. The sampling probe is provided on the pipeline at the outlet of the cooling system. The UV online detector is used to detect the sample delivered by the sampling probe and feedback the detection data to the control system to adjust the flow rate of the initial material flow. The UV online detector is connected to the control system: the preheating system is connected to the cooling system for recovering the waste heat of the cooling system after exchanging, and the preheating system is connected to the heat source system for supplying heat to the heat source system to preheat the material flow.

Furthermore, the first flushing flow system is a flushing flow module, which includes a flushing pump, a cleaning liquid storage device, a cleaning liquid recovery device, a cleaning liquid solvent distillation device, a buffering tank, and a recovery pump. The flushing pump, the cleaning liquid storage device, the cleaning liquid recovery device, the cleaning liquid solvent distillation device, the buffering tank, and the recovery pump are sequentially connected in series through pipelines. The outlet of the flushing pump is connected to the first pipeline at the outlet of the material conveying system, and the inlet of the recovery pump is connected to the outlet of the modular reaction apparatus system or the sewage discharge port of the cooling system.

Furthermore, the second flushing flow system is a flushing flow module, which includes a flushing pump, a cleaning liquid storage device, a cleaning liquid recovery device, a cleaning liquid solvent distillation device, a buffering tank, and a recovery pump. The flushing pump, the cleaning liquid storage device, the cleaning liquid recovery device, the cleaning liquid solvent distillation device, the buffering tank, and the recovery pump are sequentially connected in series through pipelines;
The outlet of the cooling system is connected to the extraction device, and the extraction device, the separation device, and the reaction product distillation device are sequentially connected;
The outlet of the flushing pump is connected to the first flushing three-way valve of the material reaction pipeline unit, and the inlet of the recovery pump is connected to the second flushing three-way valve of the material reaction pipeline unit; or the outlet of the flushing pump is connected to the inlet of the cooling system, and the inlet of the recovery pump is connected to the outlet of the cooling system; or the outlet of the flushing pump is connected to the inlet of the extraction device, and the inlet of the recovery pump is connected to the outlet of the extraction device; or the outlet of the flushing pump is connected to the inlet of the separation device, and the inlet of the recovery pump is connected to the outlet of the separation device; or the outlet of the flushing pump is connected to the inlet of the reaction product distillation device, and the inlet of the recovery pump is connected to the outlet of the reaction product distillation device.

Furthermore, the second flushing flow system is a flushing flow module, which includes a flushing pump, a cleaning liquid storage device, a cleaning liquid recovery device, a cleaning liquid solvent distillation device, a buffering tank, and a recovery pump. The flushing pump, the cleaning liquid storage device, the cleaning liquid recovery device, the cleaning liquid solvent distillation device, the buffering tank, and the recovery pump are sequentially connected in series through pipelines;
The outlet of the cooling system is connected to the extraction device through a fourth pipeline, the extraction device is connected to the separation device through a fifth pipeline, the separation device is connected to the reaction product distillation device through a sixth pipeline, and the outlet of the reaction product distillation device is connected to a seventh pipeline;
The fourth pipeline is provided with a seventh three-way valve, which has a seventh three-way valve flushing inlet. The fifth pipeline is provided with an eighth three-way valve and a ninth three-way valve. The eighth three-way valve has an eighth three-way valve flushing outlet, and the ninth three-way valve has a ninth three-way valve flushing inlet. The sixth pipeline is provided with a tenth three-way valve and an eleventh three-way valve. The tenth three-way valve has a tenth three-way valve flushing outlet, and the eleventh three-way valve has an eleventh three-way valve flushing inlet. The seventh pipeline is provided with a twelfth three-way valve, which has a twelfth three-way valve flushing outlet;
The seventh three-way valve flushing inlet, the ninth three-way valve flushing inlet, and the eleventh three-way valve flushing inlet are respectively connected to the inlet main pipe through inlet branch pipes. The eighth three-way valve flushing outlet, the tenth three-way valve flushing outlet, and the twelfth three-way valve flushing outlet are respectively connected to the main outlet pipe through the outlet branch pipes;
The outlet of the flushing pump is connected to the inlet main pipe, and the inlet of the recovery pump is connected to the outlet main pipe.

Furthermore, the flushing flow module further comprises a heating device for heating the liquid inside the cleaning liquid storage device. The heating device is connected to the cleaning liquid storage device. The cooling system is a cooling tank, which is a slender tank body with a sandwich layer. The cooling tank is provided with cooling coils inside. The upper portion of the waist of the tank body is provided with an overflow port, the lower portion of the waist is provided with a timed discharge port, and the bottom is an inverted conical discharge port. The tank body is provided with a back pressure valve. The tank body is further provided with a safety valve, a pressure sensor, and a temperature sensor.

In the other aspect, the present invention further provides a production process for continuously producing 5-hydroxymethylfurfural, comprising the following steps:
A water phase material flow is obtained by mixing sugar biomass, a solvent, an adjuvant, and an acid catalyst as raw materials uniformly;
5-hydroxymethylfurfural is obtained by preheating the water phase material flow and delivering it into a multi-stage reaction apparatus, and cooling and separating the reaction product.

Furthermore, the raw material further comprises an organic material flow, and the organic material flow and the water phase material flow are separately pumped into a multi-stage reaction apparatus through different pumps. The preheating temperature is 60 °C -90 °C, and the operating temperature of the multi-stage reaction apparatus is 120 °C -160 °C. The concentration of the sugar biomass is 10-400g/L, and the sugar biomass is at least one of glucose, fructose, sucrose, maltose, and fructose syrup. The auxiliary agent is at least one of polyethylene glycol, choline chloride, and ionic liquid. The organic solvent of the organic phase is at least one of dimethyl carbonate, diethyl carbonate, 4-methyl-2-pentanone, tetrahydrofuran, and butanol.

### (3) Beneficial effects

In summary, the above technical solution of the present invention has the following advantages:
The present invention takes into account both pure water phase and two-phase systems, adopts fully automated program to control the production process, is provided with online monitoring apparatus, and can monitor the HMF production situation in real time. When there are problems with the system temperature, pressure, and yield, corresponding solutions can be initiated by itself, saving manpower.

In addition, the present invention effectively solves the problem of the black rot easily blocking pipes caused by the generation of a large amount of humins in the actual 5-hydroxymethylfurfural production process. On the one hand, auxiliary agents are added on the raw material end to suppress the aggregation of the black rot and prevent the black rot from aggregating into blocks and causing blockage. On the other hand, modular design of the reaction apparatus and a tank body design in the cooling process can effectively prevent the precipitation of the black rot in the cooling process from blocking the pipeline. Even if the pipeline is blocked, the blocked module can be quickly replaced, and the blocked unit can be separately connected to a cleaning device for flushing, saving downtime for maintenance and not delaying factory production. As can be seen therefrom that the present device has broad development prospects for the large-scale industrial production of 5-hydroxymethylfurfural in the future.

### BRIEF DESCRIPTION OF THE DRAWINGS

Fig. 1 is a schematic diagram of the preparation device of Example 1 of the present invention filing the nitrogen to maintain the pressure of the system.
Fig. 2 is a schematic diagram of the preparation device of Example 2 of the present invention maintaining the pressure of the system using a liquid back pressure valve.
Fig. 3 is a schematic diagram of the overall cleaning structure of the modular reaction apparatus and cooling system according to the present invention.
Fig. 4 is a schematic diagram of the connection structure for separately cleaning the extraction device according to the present invention.
Fig. 5 is a schematic diagram of the connection structure for cleaning respective apparatus separately according to the present invention.

### Reference signs:

1- Water phase pump; 2- Organic pump; 3- Flushing pump; 4- Heat exchanger; 5- First three-way valve; 6- First stage reaction apparatus; 7- Second three-way valve; 8- Third three-way valve; 9-Second stage reaction apparatus; 10- Fourth three-way valve; 11- Fifth three-way valve; 12- Third stage reaction apparatus; 13- Sixth three-way valve; 14- Cooling tank; 15- Back pressure valve; 16- First three-way valve flushing inlet; 17- Third three-way valve flushing inlet; 18- Fifth three-way valve flushing inlet; 19- Second three-way valve flushing outlet (inlet); 20- Fourth three-way valve flushing outlet (inlet); 21- Sixth three-way valve flushing outlet (inlet); 22- Heat source inlet of first stage reaction apparatus; 23- Heat source inlet of second stage reaction apparatus; 24- Heat source inlet of third stage reaction apparatus; 25- Nitrogen filling port of the cooling tank; 26- Sewage discharge port of the cooling tank; 27- Cooling liquid discharge port of the cooling tank; 28. Seventh three-way valve flushing inlet; 29. Extraction device; 30. Eighth three-way valve flushing outlet; 31. Ninth three-way valve flushing inlet; 32. Separation device; 33. Tenth three-way valve flushing outlet; 34. Eleventh three-way valve flushing inlet; 35. Reaction product distillation device; 36. Twelfth three-way valve flushing outlet; 38. Cleaning liquid storage device; 39. Cleaning liquid recovery device; 40. Cleaning liquid solvent distillation device; 41. Buffering tank; 42. Recovery pump; 100. Modular reaction apparatus.

### DETAILED DESCRIPTION

Below, a further detailed description of the embodiments of the present invention will be provided in conjunction with the accompanying drawings and examples. The detailed description and accompanying drawings of the following embodiments are used to exemplarily illustrate the principles of the present invention, but cannot be used to limit the scope of the present invention, that is, the present invention is not limited to the described embodiments.

In order to better understand the above object, features, and advantages of the present invention, the solution of the present invention will be further described below. It should be noted that the embodiments and features of the present invention can be combined with each other without conflict.

Many specific details are set forth in the following description to facilitate a full understanding of the invention, but the present invention may also be implemented in other ways different from those described herein. Obviously, the embodiments described in the description are only a part of the embodiments of the present invention, not all of them.

The preferred embodiments of the present invention will be described in detail below in conjunction with the embodiments. It should be understood that the following embodiments are provided for illustrative purposes only and are not intended to limit the scope of the present invention. The skilled person in the art may make various modifications and substitutions to the present invention without departing from the object and spirit of the present invention.

The experimental methods used in the following examples are conventional methods unless otherwise specified.

The materials, reagents, etc. used in the following embodiments can be purchased commercially unless otherwise specified.

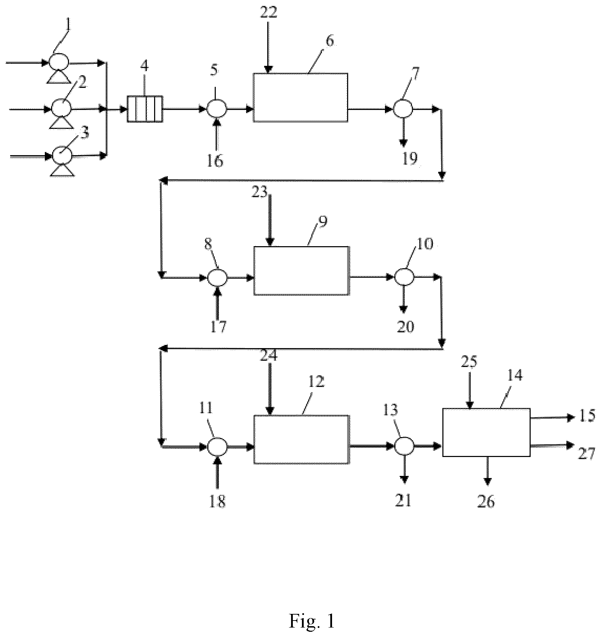

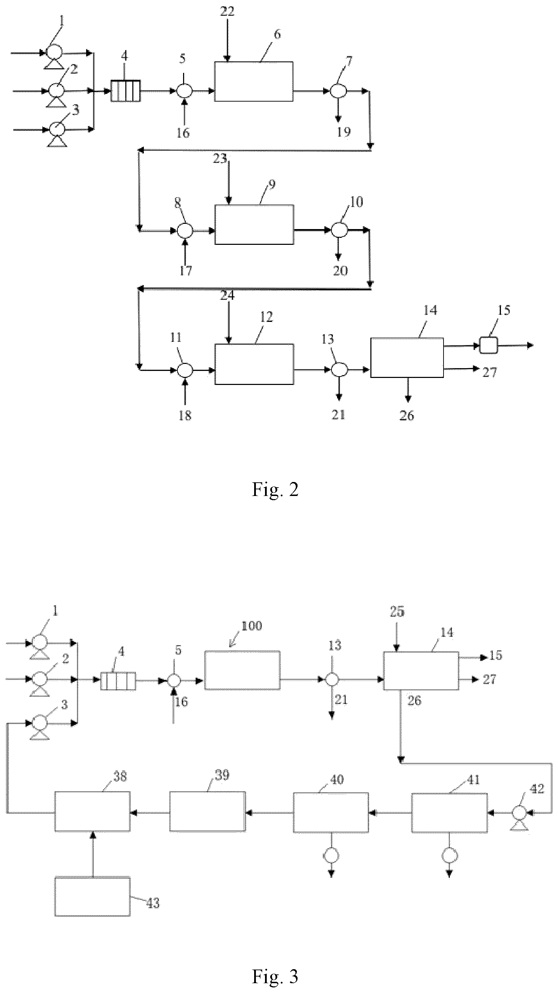

As shown in Figs. 1 and 2, in a first aspect, the present invention provides a device for continuously producing 5-hydroxymethylfurfural, which comprises:
a material conveying system including a water phase material flow conveying unit for transporting the water phase material flow;
a first flushing flow system for cleaning during blockage and discharge, the first flushing flow system and the water phase material flow conveying unit being connected to the outlet of the material conveying system respectively;
a heat source system for heating the material, the heat source system being connected to the outlet of the material conveying system through a first pipeline;
a modular reaction apparatus system connected to the outlet of the heat source system through a second pipeline, the modular reaction apparatus system being built-in with a material reaction pipeline unit, a temperature sensing control unit, and a pressure sensing control unit;
a cooling system for cooling the products in the modular reaction apparatus system, the cooling system being connected to the outlet of the modular reaction apparatus system through a third pipeline;
a control system, the water phase material flow conveying unit, the first flushing flow system, the heat source system, the temperature sensing control unit, the pressure sensing control unit, and the cooling system are connected to the control system respectively, for intelligent control of feeding, apparatus automatic flushing, temperature, pressure, emergency start, and emergency stop.

Furthermore, the material conveying system further comprises an organic phase material flow conveying unit for conveying the organic material flow. The organic phase material flow conveying unit is connected to the outlet of the material conveying system.

Preferably, the inorganic phase material flow conveying unit is a water phase pump, the organic phase material flow conveying unit is an organic pump, the first flushing flow system is a flushing pump, and more preferably, the water phase pump, organic pump, and flushing pump are all high-pressure pumps.

It should be noted that when the conveyed material only has a water phase material flow, the water phase pump operates and the organic pump is turned off. When the conveyed materials have both a water phase material flow and an organic material flow, both the water phase pump and the organic pump operate. When the system is blocked and needs to be flushed, the flushing pump is turned on, and when the system operates normally, the flushing pump is turned off. When the system detects that the pressure exceeds the maximum limit of the reaction pressure, the water phase pump and the organic phase pump are turned off, and the flushing pump is turned on to enter the pipeline flushing mode to prevent further accumulation of the black rot in the system.

Furthermore, the modular reaction apparatus system comprises a plurality of modular reaction apparatuses. The temperature sensing control unit is a temperature sensor, and the pressure sensing control unit is a pressure sensor. The modular reaction apparatus includes a material reaction pipeline unit, a temperature sensor, and a pressure sensor that are built-in. The interior of the material reaction pipeline unit is entirely lined with polytetrafluoroethylene inner pipes, and the interior of the material reaction pipeline unit is provided with a temperature sensor and a pressure sensor, which are respectively connected to the control system. The material reaction pipeline unit is provided with a heating element.

It should be noted that modular design of the reaction apparatus refers to the modular design of the traditional fixed bed catalyst loading area or the material countercurrent (downstream) reaction area, that is, the core area of the dehydration reaction.

Preferably, the plurality of modular reaction apparatuses can be sequentially connected in series or parallel according to actual production capacity requirements.

Preferably, the material reaction pipeline system comprises a reaction coil, a three-way valve of inflow pipeline, a feed pump, and a three-way valve of the outflow pipeline.

In order to better prevent the system from blockage, the layout of the pipelines inside the modular reaction apparatus is preferably in the form of coils, and the liquid is transported from top to bottom.

In order to suppress the black rot from hanging on the wall of the pipeline or from blocking the pipeline and improve the acid and corrosion resistance of the pipeline, the interior of the reaction pipeline is entirely lined with polytetrafluoroethylene inner pipes, and is built-in with a plurality of temperature and pressure sensors to feedback a real-time status of the material inside the pipeline so that a timely adjustment can be performed if any abnormal situation occurs.

Furthermore, the cooling system is a cooling tank, which is a slender tank body with a sandwich layer. The cooling tank is provided with cooling coils inside. The upper portion of the waist of the tank body is provided with an overflow port, the lower portion of the waist is provided with a timed discharge port, and the bottom is an inverted conical discharge port. The tank body is provided with a back pressure valve. The tank body is further provided with a safety valve, a pressure sensor, and a temperature sensor.

It should be noted that according to the different structures of the cooling tank, it can be divided into two forms. One form is to use nitrogen pressurization to ensure a certain pressure of the system and timed discharge. The other form is that the liquid overflows after cooling and passes through a back pressure valve so as to ensure a certain pressure of the system.

Furthermore, a second flushing flow system is further included, and the modular reaction apparatus further comprises a first flushing three-way valve and a second flushing three-way valve. The first flushing three-way valve and the second flushing three-way valve are respectively disposed at the position near the inlet of the material reaction pipeline unit and the position near the outlet of the material reaction pipeline unit. The first flushing three-way valve is connected to the second flushing flow system, and the first flushing three-way valve and the second flushing three-way valve are respectively connected to the control system.

The three-way valves at the inlet and outlet of the material reaction pipeline unit are used for conveying the material flow during normal operation of the reaction apparatus, and for introducing the flushing liquid when the device is blocked.

Furthermore, the first flushing flow system is a flushing pump, or the first flushing flow system is a flushing flow module. The flushing flow module includes a flushing pump, a cleaning liquid storage device, a cleaning liquid recovery device, a cleaning liquid solvent distillation device, a buffering tank, and a recovery pump. The flushing pump, the cleaning liquid storage device, the cleaning liquid recovery device, the cleaning liquid solvent distillation device, the buffering tank, and the recovery pump are sequentially connected in series through pipelines. The outlet of the flushing pump is connected to the first pipeline at the outlet of the material conveying system, and the inlet of the recovery pump is connected to the outlet of the modular reaction apparatus system or the sewage discharge port of the cooling system. The recovery pump is used to transport the extracted cleaning liquid to the buffering tank, where it undergoes initial filtration and slag discharge.

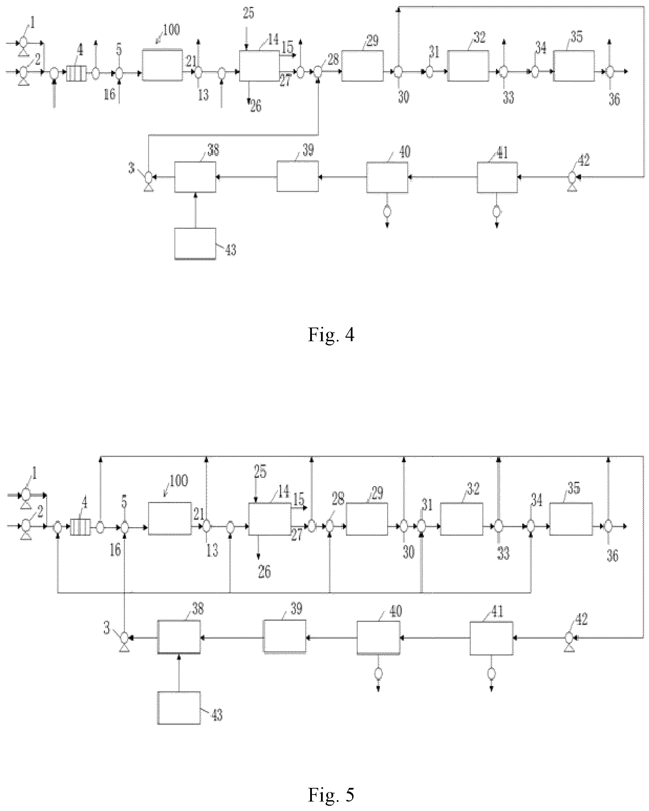

As shown in Fig. 4, the second flushing flow system is a flushing flow module. The flushing flow module includes a flushing pump, a cleaning liquid storage device, a cleaning liquid recovery device, a cleaning liquid solvent distillation device, a buffering tank, and a recovery pump. The flushing pump, the cleaning liquid storage device, the cleaning liquid recovery device, the cleaning liquid solvent distillation device, the buffering tank, and the recovery pump are sequentially connected in series through pipelines;

The outlet of the cooling system is connected to the extraction device, and the extraction device, the separation device, and the reaction product distillation device are sequentially connected. And as described below, multiple connection configurations with the flushing pump and the recovery pump are provided:

The outlet of the flushing pump is connected to the first flushing three-way valve of the material reaction pipeline unit, and the inlet of the recovery pump is connected to the second flushing three-way valve of the material reaction pipeline unit; or the outlet of the flushing pump is connected to the inlet of the cooling system, and the inlet of the recovery pump is connected to the outlet of the cooling system; or the outlet of the flushing pump is connected to the inlet of the extraction device, and the inlet of the recovery pump is connected to the outlet of the extraction device; or the outlet of the flushing pump is connected to the inlet of the separation device, and the inlet of the recovery pump is connected to the outlet of the separation device; or the outlet of the flushing pump is connected to the inlet of the reaction product distillation device, and the inlet of the recovery pump is connected to the outlet of the reaction product distillation device.

As shown in Fig. 5, the second flushing flow system is a flushing flow module, which includes a flushing pump, a cleaning liquid storage device, a cleaning liquid recovery device, a cleaning liquid solvent distillation device, a buffering tank, and a recovery pump. The flushing pump, the cleaning liquid storage device, the cleaning liquid recovery device, the cleaning liquid solvent distillation device, the buffering tank, and the recovery pump are sequentially connected in series through pipelines;

The outlet of the cooling system is connected to the extraction device through a fourth pipeline, the extraction device is connected to the separation device through a fifth pipeline, the separation device is connected to the reaction product distillation device through a sixth pipeline, and the outlet of the reaction product distillation device is connected to a seventh pipeline;

The fourth pipeline is provided with a seventh three-way valve, which has a seventh three-way valve flushing inlet. The fifth pipeline is provided with an eighth three-way valve and a ninth three-way valve. The eighth three-way valve has an eighth three-way valve flushing outlet, and the ninth three-way valve has a ninth three-way valve flushing inlet. The sixth pipeline is provided with a tenth three-way valve and an eleventh three-way valve. The tenth three-way valve has a tenth three-way valve flushing outlet, and the eleventh three-way valve has an eleventh three-way valve flushing inlet. The seventh pipeline is provided with a twelfth three-way valve, which has a twelfth three-way valve flushing outlet.

The seventh three-way valve flushing inlet, the ninth three-way valve flushing inlet, and the eleventh three-way valve flushing inlet are respectively connected to the inlet main pipe through inlet branch pipes. The eighth three-way valve flushing outlet, the tenth three-way valve flushing outlet, and the twelfth three-way valve flushing outlet are respectively connected to the outlet main pipe through the outlet branch pipes;
The outlet of the flushing pump is connected to the inlet main pipe, and the inlet of the recovery pump is connected to the outlet main pipe.

Through the above connection configuration manner, when a certain apparatus needs to be cleaned, the valves near both sides of the apparatus are opened and the valves on both sides of other apparatuses are closed. The cleaning liquid is pumped from the flushing pump to the inlet main pipe, and then enter the apparatus through the corresponding inlet branch pipe. The cleaning waste liquid is then drawn out of the apparatus by the recovery pump to the outlet branch pipe. The cleaning waste liquid enters the outlet main pipe from the outlet branch pipe and flows back to the recovery pump from the outlet main pipe. Finally, the subsequent recover and utilization process of the cleaning waste liquid is carried out.

Furthermore, the flushing flow module further comprises a heating device for heating the liquid inside the cleaning liquid storage device. The heating device is connected to the cleaning liquid storage device. In the production process of 5-hydroxymethylfurfural, the black rot often solidifies and adheres to the surface of the apparatus, making it difficult to be removed by the cleaning liquid at ordinary temperature. To improve the cleaning effect, the black rot can be softened and dissolved by heating the cleaning liquid. Therefore, a heating device was introduced to heat the liquid inside the cleaning liquid storage device.

In the above embodiments, in order to ensure the quality and service life of the cleaning liquid, and achieve the cycling usage of the cleaning liquid, a cleaning liquid solvent distillation device and a cleaning liquid recovery device are introduced for secondary treatment of the cleaning liquid. The cleaning liquid solvent distillation device is provided with a heating assembly inside, which can remove impurities and residues from the cleaning liquid through heating and distillation operations, thereby purifying0 the cleaning liquid, and extending its service life.

Furthermore, the rear end of the timed discharge port of the cooling tank is connected to the extraction device through a fourth pipeline, the extraction device is connected to the separation device through a fifth pipeline, the separation device is connected to the reaction product distillation device through a sixth pipeline, and the outlet of the reaction product distillation device is connected to a seventh pipeline.

Due to the valves disposed on the fourth, fifth, sixth, and seventh pipelines, the extraction device, the separation device, and the reaction product distillation device can be cleaned separately.

Furthermore, an online monitoring system is further included. The online monitoring system includes an UV online detector and a sampling probe connected to each other. The sampling probe is disposed on the pipeline at the outlet of the cooling system, and the UV online detector is used to detect the sample transported by the sampling probe and feedback the detection data to the control system to adjust the flow rate of the initial material flow.

Furthermore, a preheating system is further included. The preheating system is connected to the cooling system for recovering the waste heat of the cooling system after exchanging. The preheating system is connected to the heat source system for supplying heat to the heat source system to preheat the material flow.

The heat source of the reaction apparatus comes from the steam generated by the heat source system, and the temperature is automatically controlled by adjusting the valve opening through the built-in temperature control sensor. The pipeline connections between the reaction apparatuses are insulated with an insulation cotton.

According to the disclosed example, the cleaning liquid in the cleaning liquid storage device includes various cleaning agents with strong solubility for the black rot generated in the HMF production process. The composition of the cleaning liquid includes not only conventional commercially available detergents and oil fume descaling agents that have good removal effects on oil fume, but also organic solvents. Therefore, at time of selecting the cleaning liquid, it can be one or a combination of detergent, oil fume descaling agent, or organic solvent.

In this embodiment, the organic solvent is at least one of fluorocarbon organic solvents, halogenated hydrocarbon organic solvents, alcohol organic solvents, amide organic solvents, ester organic solvents, furan organic solvents, and other organic solvents.

The organic solvent is at least one of dichloromethane, chloroform, carbon tetrachloride, dichlorodifluoromethane, monochlorotrichloromethane, trichlorotrifluoroethane, tetrachloroethylene, methanol, ethanol, isopropanol, n-butanol, ethylene glycol, polyethylene glycol, glycerol, N, N-dimethylformamide, N, N-dimethylacetamide, methyl acetate, ethyl acetate, tetrahydrofuran, 2-methyltetrahydrofuran, 4-methyl-2-pentanone, dimethyl carbonate, diethyl carbonate, acetonitrile, dimethyl sulfoxide, N-methylpyrrolidone, acetone, etc.

When the cleaning and slag removal system operates, the mixed cleaning liquid is atomized and loaded into the modular reaction apparatus to be cleaned under certain temperature and pressure conditions, and the process is repeated for 5-120 minutes. Then, the apparatus can be put back into use after being washed with organic solvent and water in sequence respectively.

In this embodiment, the certain temperature includes room temperature -150 °C. The certain pressure includes 0.1-2MPa.

In the other aspect, the present invention further provides a production process for continuously producing 5-hydroxymethylfurfural, comprising:
The water phase material flow is obtained by mixing sugar biomass, a solvent, an adjuvant, and an acid catalyst as raw materials uniformly.

The water phase material flow is preheated and is delivered to a multi-stage reaction apparatus, and 5-hydroxymethylfurfural is obtained by cooling and separating the reaction product.

Furthermore, the raw material further includes an organic material flow, and the organic material flow and the water phase material flow are separately pumped into a multi-stage reaction apparatus through different pumps. The preheating temperature is 60 °C -90 °C, and the operating temperature of the multi-stage reaction apparatus is 120 °C -160 °C.

Furthermore, the concentration of the sugar biomass is 10 g/L to 400 g/L, and the sugar biomass is at least one of glucose, fructose, sucrose, maltose, and fructose syrup. The auxiliary agent is at least one of polyethylene glycol, choline chloride, and ionic liquid. The organic solvent of the organic phase is at least one of dimethyl carbonate, diethyl carbonate, 4-methyl-2-pentanone, tetrahydrofuran, and butanol.

It should be noted that the function of the auxiliary agent is to enhance the selectivity of 5-hydroxymethylfurfural and inhibit the production of the black rot.

Preferably, the solvent is water.

Preferably, the acid catalyst is a homogeneous acid, including inorganic acid and organic acid. More preferably, the inorganic acid is at least one of sulfuric acid, hydrochloric acid, and phosphoric acid, and the organic acid is at least one of methanesulfonic acid, p-toluenesulfonic acid, tartaric acid, and citric acid.

Example 1

As shown in Fig. 1, the preparation method of 5-hydroxymethylfurfural in the two-phase system provided by the present invention is as follows:
Fructose 60kg, water 400kg, phosphoric acid 1.8kg and polyethylene glycol 4000 20kg were weighed, stirred and dissolved, and then labeled as the water phase raw material. The dimethyl carbonate solvent is the organic phase.

Reaction is conducted with the apparatus shown in Fig. 1. During normal operation, nitrogen is loaded and the system pressure is maintained above 0.8 MPa. The heat source steam keeps the system between 140 °C and 150 °C. The organic pump 2 pumps the organic phase dimethyl carbonate solvent at a rate of 1 L/min, and the water phase pump 1 pumps the water phase raw material at a rate of 0.5 L/min. The flushing pump 3 is turned off, and the organic and water phases both are mixed and passed through the heat source system, namely the heat exchanger 4, and first three-way valve 5 sequentially into the first stage reaction apparatus 6. The material obtained from the first stage reaction apparatus 6 enters the second stage reaction apparatus 9 through the second three-way valve 7 and the third three-way valve 8 sequentially, and the material obtained from the second stage reaction apparatus 9 enters the third stage reaction apparatus 12 through the fourth three-way valve 10 and the fifth three-way valve 11 sequentially. The material obtained from the third stage reaction apparatus 12 enters the cooling tank 14 through the sixth three-way valve 13.

After entering the cooling tank 14, the material obtained from the reaction is cooled to 60 °C -80 °C in the cooling tank. A nitrogen filling port 25 is provided on the upper portion of the side surface of the cooling tank, which is filled with nitrogen to maintain a certain system pressure. The system pressure is automatically controlled. A gas back pressure valve 15 is provided at the outlet of the cooling tank. A cooling liquid discharge port 27 is provided on the lower portion of the side surface of the cooling tank and the cooling liquid discharge port 27 is a timed discharge port. After discharge, the material flows to the extraction device 29, the separation device 32, and the reaction product distillation device 35. The extraction device 29 is used to extract the reactants, the separation device 32 is used to further separate the target product and other impurities, and the reaction product distillation device 35 is used for the purification of the products. Each device plays a different role throughout the entire preparation process to ensure the final acquisition of high-purity 5-hydroxymethylfurfural. A sewage discharge port 26 mainly for discharging the deposited black rot is provided at the bottom of the cooling tank.

When the apparatus operates normally, valves of the three-way valve flushing inlets 16, 17, and 18 in front of the reaction apparatus are in closed state, and valves of the three-way valve flushing outlets (inlets) 19, 20, and 21 behind the reaction apparatus are in closed state. Reaction apparatuses 6, 9, and 12 are respectively provided with steam heat source inlets 22, 23, and 24. When a micro blockage occurs in a certain one of the reaction apparatuses that are connected in series, the same module can be used to replace the reaction apparatus. Assuming that the second stage reaction apparatus 9 needs to be replaced, it only needs to connect one pipe corresponding to the tee of the feed inlet of the backup reaction apparatus to the second three-way valve flushing inlet 19, connect one pipe corresponding to the tee of the discharge outlet to the fifth three-way valve 11 and switch the corresponding valves after the completion of the connection, to ensure smooth operation of the reaction apparatus. And it is also very convenient to clean the second stage reaction apparatus 9. It only needs to connect the pipeline of the second three-way valve flush inlet 17 to the flushing pump correspondingly, close the feeding valve of the third three-way valve 8 and the discharging valve of the fourth three-way valve 10, open the pipe of the third three-way valve flushing inlet 17, and the valve of the fourth three-way valve flushing outlet 20 so that the second stage reaction apparatus 9 can be cleaned separately and used as a backup.

By detecting and analyzing the reaction mixture, the molar yield of 5-hydroxymethylfurfural was measured as 50%.

### Example 2

As shown in Fig. 2, the preparation method of pure water phase 5-hydroxymethylfurfural provided by the present invention is as follows:
Fructose 60kg, water 400kg, phosphoric acid 1.8kg and polyethylene glycol 4000 20kg were weighed, stirred and dissolved, and then labeled as water phase raw material..

Reaction is conducted with the apparatus shown in Fig. 2. During normal operation, the back pressure valve 15 adjusts the pressure to 0.8Mpa, and the heat source steam maintains the system between 140 °C and 150 °C. The water phase pump 1 pumps in the water phase raw material at a rate of 1.5L/min, and the organic pump 2 and the flushing pump 3 are turned off. The water phase material flow enters the first stage reaction apparatus 6 through the heat exchanger 4 and the first three-way valve 5 in sequence. The material obtained from the first stage reaction apparatus 6 enters the second stage reaction apparatus 9 through the second three-way valve 7 and the third three-way valve 8 in sequence. The material obtained from the second stage reaction apparatus 9 enters the third stage reaction apparatus 12 through the fourth three-way valve 10 and the fifth three-way valve 11 in sequence. The material obtained from the third stage reaction apparatus 12 enters the cooling tank 14 through the sixth three-way valve 13.

After entering cooling tank 14, the material is cooled to 60 °C -80 °C in the cooling tank. The upper portion of the side surface of the cooling tank is provided with an overflow port. The upper layer of cooling liquid flows through the back pressure valve 15 to the storage tank by means of overflow. The discharge port 27 on the lower portion of the side surface of the cooling tank is an emergency discharge port, which is closed during normal operation. A sewage discharge port 26 mainly for discharging the deposited black rot is further disclosed at the bottom of the cooling tank.

When the apparatus operates normally, valves of the three-way valve flushing inlets 16, 17, and 18 in front of the reaction apparatus are in closed state, and valves of the three-way valve flushing outlets (inlets) 19, 20, and 21 behind the reaction apparatus are in closed state. The reaction apparatuses 6, 9, and 12 are respectively provided with steam heat source inlets 22, 23, and 24. When a micro blockage occurs in a certain one of the reaction apparatuses that are connected in series, the same module can be used to replace the reaction apparatus. Assuming that the secondary reaction apparatus 9 needs to be replaced, it only needs to connect one pipe corresponding to the tee of the feed inlet of the backup reaction apparatus to the second three-way valve flushing inlet 19, connect one pipe corresponding to the tee of the discharge outlet to the fifth three-way valve 11 and switch the corresponding valves after the completion of the connection, to ensure smooth operation of the reaction apparatus. And it is also very convenient to clean the second stage reaction apparatus 9. It only needs to connect the pipeline of the second three-way valve flush inlet 17 to the flushing pump correspondingly, close the feeding valve of the third three-way valve 8 and the discharging valve of the fourth three-way valve 10, open the pipe of the third three-way valve flushing inlet 17, and the valve of the fourth three-way valve flushing outlet 20 so that the second stage reaction apparatus 9 can be cleaned separately and used as a backup.

By detecting and analyzing the reaction mixture, the molar yield of 5-hydroxymethylfurfural was measured as 43%.

The cleaning of the black rot includes the overall cleaning of the device and the individual cleaning of a certain apparatus in the device. The following will provide a detailed explanation of the process of overall cleaning and individual cleaning of a certain apparatus.

Fig. 3 shows the overall cleaning of a plurality of modular reaction apparatus 100 (i.e., the first stage reaction apparatus 6, the second stage reaction apparatus 9, and the third stage reaction apparatus 12). During the specific cleaning process, firstly the water is atomized and sprayed by the flushing pump 3 at a certain pressure and enters the heat exchanger 4, the first three-way valve 5, and the reaction apparatuses 6, 9, and 12 to wash off most of the reaction liquid and block shaped black rot. The waste liquid then flows out of the sixth three-way valve flushing outlet (inlet) 21 into the buffering tank 41 and the cleaning liquid solvent distillation device 40 for water discharge and slag discharge by the recovery pump 42. The filtered cleaning liquid enters the cleaning liquid recovery device 39, and then enters the cleaning liquid storage device 38 from the cleaning liquid recovery device 39. After that, organic solvents such as amides, ketones, alcohols, or esters is added, or a small amount of detergent 0.1-5% is added, or oil fume descaling agent 0.1-5% is added further. After being stirred and mixed uniformly, the mixed liquid is heated by the heating device 43, followed by high-pressure cleaning. The process is repeated for 5-120 minutes. Afterwards, a single type of organic solvent is added, and is heated to around 100 °C, followed by high-pressure cleaning, and the process is repeated for 5-120 minutes. Finally, high-pressure cleaning is conducted with tap water and the process is repeated for 5-120 minutes. After the system is restored, the system can continue to be used. In this process, organic solvents are classified and collected, respectively recovered to the cleaning liquid recovery device 39, and then compounded for use.

Figs. 4 and 5 show the separate cleaning of the extraction device 29, the separation device 32, and the reaction product distillation device 35. When the extraction device 29 is cleaned separately, the seventh three-way valve flushing inlet 28 and the eighth three-way valve flushing outlet 30 are opened, valves of the three-way valve flushing inlets 16, 17, and 18 before the reaction apparatus and valves of the three-way valve flushing outlets (inlets) 19, 20, and 21 behind the reaction apparatus are closed, the ninth three-way valve flushing inlet 31, the tenth three-way valve flushing outlet 33, the eleventh three-way valve flushing inlet 34, and the twelfth three-way valve flushing outlet 36 are closed, and the flushing pump 3 is started. Firstly the water is atomized and sprayed by the flushing pump 3 at a certain pressure and enters the extraction device 29 to wash off most of the reaction liquid and block shaped black rot in the extraction device 29. The waste liquid then flows out of the eighth three-way valve flushing outlet 30 21 into the buffering tank 41 and the cleaning liquid solvent distillation device 40 for water discharge and slag discharge by the recovery pump 42. The filtered cleaning liquid enters the cleaning liquid recovery device 39, and then enters the cleaning liquid storage device 38 from the cleaning liquid recovery device 39. After that, organic solvents such as amides, ketones, alcohols, or esters is added, or a small amount of detergent 0.1-5% is added, or oil fume descaling agent 0.1-5% is added further. After being stirred and mixed uniformly, the mixed liquid is heated by the heating device 43, followed by high-pressure cleaning. The process is repeated for 5-120 minutes. Afterwards, a single type of organic solvent is added, and is heated to around 100 °C, followed by high-pressure cleaning, and the process is repeated for 5-120 minutes. Finally, high-pressure cleaning is conducted with tap water and the process is repeated for 5-120 minutes. After the system is restored, the system can continue to be used. In this process, organic solvents are classified and collected, respectively recovered to the cleaning liquid recovery device 39, and then compounded for use

The steps for separately cleaning the separation device 32 and the reaction product distillation device 35 are the same as those for cleaning the extraction device 29, and will not be repeated. In addition, the present application can also clean the heat exchanger 4, which is connected to the inlet and outlet main pipes through pipelines.

It should be clarified that the present invention is not limited to the specific steps and structures described above and shown in the figures. And, for the sake of simplicity, detailed descriptions of known methods and techniques are omitted here.

The above description is only an embodiment of the present application and is not limited to the present application. For those skilled in the art, various modifications and variations can be made to this application without departing from the scope of the present invention. Any modifications, equivalent substitutions, improvements, etc. made within the spirit and principles of this application shall be included within the scope of the claims of this application.

## Claims

1. A device for continuously producing 5-hydroxymethylfurfural, **characterized by** comprising:
a material conveying system comprising a water phase material flow conveying unit for conveying a water phase material flow;
a first flushing flow system for cleaning during blockage and discharge, the first flushing flow system and the water phase material flow conveying unit being connected to an outlet of the material conveying system respectively;
a heat source system for heating the material, the heat source system being connected to an outlet of the material conveying system through a first pipeline;
a modular reaction apparatus system connected to an outlet of the heat source system through a second pipeline, the modular reaction apparatus system being built-in with a material reaction pipeline unit, a temperature sensing control unit, and a pressure sensing control unit;
a cooling system for cooling products in the modular reaction apparatus system, the cooling system being connected to an outlet of the modular reaction apparatus system through a third pipeline;
a control system, the water phase material flow conveying unit, the first flushing flow system, the heat source system, the temperature sensing control unit, the pressure sensing control unit, and the cooling system are connected to the control system respectively, for intelligent control of feeding, apparatus automatic flushing, temperature, pressure, emergency start, and emergency stop.

2. The device according to claim 1, **characterized in that** the material conveying system further comprises an organic phase material flow conveying unit for conveying an organic material flow, and the organic phase material flow conveying unit is connected to the outlet of the material conveying system.

3. The device according to claim 1, **characterized in that** the modular reaction apparatus system comprises a plurality of modular reaction apparatuses, the temperature sensing control unit is a temperature sensor, the pressure sensing control unit is a pressure sensor, the modular reaction apparatus comprises a material reaction pipeline unit, a temperature sensor, and a pressure sensor that are built-in, the interior of the material reaction pipeline unit is entirely lined with a polytetrafluoroethylene inner pipe, the interior of the material reaction pipeline unit is built-in with a temperature sensor and a pressure sensor, which are connected to the control system respectively, and the material reaction pipeline unit is provided with a heating element connected to the control system.

4. The device according to claim 3, **characterized in that** the device further comprises a second flushing flow system, and the plurality of modular reaction apparatuses are sequentially arranged in series or parallel, the modular reaction apparatus further comprises a first flushing three-way valve and a second flushing three-way valve, the first flushing three-way valve and the second flushing three-way valve are respectively provided at the position near an inlet of the material reaction pipeline unit and the position near an outlet of the material reaction pipeline unit, the first flushing three-way valve is connected to the second flushing flow system, and the first flushing three-way valve and the second flushing three-way valve are respectively connected to the control system.

5. The device according to claim 1, **characterized in that** the device further comprises an online monitoring system and a preheating system, the online monitoring system comprises a UV online detector and a sampling probe connected to each other, the sampling probe is provided on a pipeline at an outlet of the cooling system, the UV online detector is used to detect a sample delivered by the sampling probe and feedback detection data to the control system to adjust initial material flow rate, the UV online detector is connected to the control system: the preheating system is connected to the cooling system for recovering the waste heat of the cooling system after exchanging, and the preheating system is connected to the heat source system for supplying heat to the heat source system to preheat the material flow.

6. The device according to claim 1, **characterized in that** the first flushing flow system is a flushing flow module, which comprises a flushing pump, a cleaning liquid storage device, a cleaning liquid recovery device, a cleaning liquid solvent distillation device, a buffering tank, and a recovery pump, wherein the flushing pump, the cleaning liquid storage device, the cleaning liquid recovery device, the cleaning liquid solvent distillation device, the buffering tank, and the recovery pump are sequentially connected in series through pipelines, an outlet of the flushing pump is connected to a first pipeline at the outlet of the material conveying system, and an inlet of the recovery pump is connected to the outlet of the modular reaction apparatus system or a sewage discharge port of the cooling system.

7. The device according to claim 4, **characterized in that** the second flushing flow system is a flushing flow module, which comprises a flushing pump, a cleaning liquid storage device, a cleaning liquid recovery device, a cleaning liquid solvent distillation device, a buffering tank, and a recovery pump, wherein the flushing pump, the cleaning liquid storage device, the cleaning liquid recovery device, the cleaning liquid solvent distillation device, the buffering tank, and the recovery pump are sequentially connected in series through pipelines;
an outlet of the cooling system is connected to an extraction device, and the extraction device, a separation device, and a reaction product distillation device are connected sequentially;
an outlet of the flushing pump is connected to the first flushing three-way valve of the material reaction pipeline unit, and an inlet of the recovery pump is connected to the second flushing three-way valve of the material reaction pipeline unit; or the outlet of the flushing pump is connected to an inlet of the cooling system, and the inlet of the recovery pump is connected to the outlet of the cooling system; or the outlet of the flushing pump is connected to an inlet of the extraction device, and the inlet of the recovery pump is connected to an outlet of the extraction device; or the outlet of the flushing pump is connected to an inlet of the separation device, and the inlet of the recovery pump is connected to an outlet of the separation device; or the outlet of the flushing pump is connected to an inlet of the reaction product distillation device, and the inlet of the recovery pump is connected to an outlet of the reaction product distillation device.

8. The device according to claim 4, **characterized in that** the second flushing flow system is a flushing flow module, which comprises a flushing pump, a cleaning liquid storage device, a cleaning liquid recovery device, a cleaning liquid solvent distillation device, a buffering tank, and a recovery pump, wherein the flushing pump, the cleaning liquid storage device, the cleaning liquid recovery device, the cleaning liquid solvent distillation device, the buffering tank, and the recovery pump are sequentially connected in series through pipelines;
an outlet of the cooling system is connected to an extraction device through a fourth pipeline, the extraction device is connected to a separation device through a fifth pipeline, the separation device is connected to a reaction product distillation device through a sixth pipeline, and an outlet of the reaction product distillation device is connected to a seventh pipeline;
the fourth pipeline is provided with a seventh three-way valve, which has a seventh three-way valve flushing inlet; the fifth pipeline is provided with an eighth three-way valve and a ninth three-way valve, the eighth three-way valve has an eighth three-way valve flushing outlet, and the ninth three-way valve has a ninth three-way valve flushing inlet; the sixth pipeline is provided with a tenth three-way valve and an eleventh three-way valve, the tenth three-way valve has a tenth three-way valve flushing outlet, and the eleventh three-way valve has an eleventh three-way valve flushing inlet; the seventh pipeline is provided with a twelfth three-way valve, which has a twelfth three-way valve flushing outlet;
the seventh three-way valve flushing inlet, the ninth three-way valve flushing inlet, and the eleventh three-way valve flushing inlet are respectively connected to an inlet main pipe through an inlet branch pipe; the eighth three-way valve flushing outlet, the tenth three-way valve flushing outlet, and the twelfth three-way valve flushing outlet are respectively connected to an outlet main pipe through an outlet branch pipe;
an outlet of the flushing pump is connected to the inlet main pipe, and an inlet of the recovery pump is connected to the outlet main pipe.

9. The device according to any one of claims 6-8, **characterized in that** the flushing flow module further comprises a heating device for heating the liquid inside the cleaning liquid storage device, the heating device is connected to the cleaning liquid storage device; the cooling system is a cooling tank, which is a slender tank body with a sandwich layer, the interior of the cooling tank is provided with cooling coils; an upper portion of a waist of the tank body is provided with an overflow port, a lower portion of the waist is provided with a timed discharge port, and the bottom is an inverted conical discharge port; the tank body is provided with a back pressure valve; the tank body is further provided with a safety valve, a pressure sensor, and a temperature sensor.

10. A production process for continuously producing 5-hydroxymethylfurfural, **characterized by** comprising the following steps:
a water phase material flow is obtained by mixing sugar biomass, a solvent, an adjuvant, and an acid catalyst as raw material uniformly;
5-hydroxymethylfurfural is obtained by preheating the water phase material flow and delivering it into a multi-stage reaction apparatus, and cooling and separating the reaction product.

11. The production process according to claim 10, **characterized in that** the raw material further comprises an organic material flow, and the organic material flow and the water phase material flow are respectively pumped into the multi-stage reaction apparatus through different pumps; the preheating temperature is 60 °C -90 °C, and the operating temperature of the multi-stage reaction apparatus is 120 °C -160 °C; the concentration of the sugar biomass is 10-400g/L, and the sugar biomass is at least one of glucose, fructose, sucrose, maltose, and fructose syrup; the auxiliary agent is at least one of polyethylene glycol, choline chloride, and ionic liquid; the organic solvent of the organic phase is at least one of dimethyl carbonate, diethyl carbonate, 4-methyl-2-pentanone, tetrahydrofuran, and butanol.
